Europäisches Patentamt

European Patent Office

Office européen des brevets

(19)

(11) Publication number: **0 122 749**

**B1**

(12) **EUROPEAN PATENT SPECIFICATION**

(45) Date of publication of patent specification: **20.04.88**

(51) Int. Cl.⁴: **G 01 N 33/06**, G 01 N 21/35

(21) Application number: **84302282.3**

(22) Date of filing: **03.04.84**

(54) **Measurement of fat.**

(30) Priority: **05.04.83 GB 8309153**

(43) Date of publication of application:
**24.10.84 Bulletin 84/43**

(45) Publication of the grant of the patent:
**20.04.88 Bulletin 88/16**

(84) Designated Contracting States:
**AT BE CH DE FR GB IT LI LU NL SE**

(56) References cited:
**EP-A-0 012 492**
**DE-A-1 297 896**
**FR-A-2 050 525**
**US-A-3 066 570**
**US-A-3 839 633**
**US-A-4 337 396**

(73) Proprietor: **SHIELDS INSTRUMENTS LIMITED**
**Wheldrake**
**York Y04 6NA (GB)**

(72) Inventor: **Shields, John**
**c/o Shields Instruments Ltd. Wheldrake**
**York, Y04 6NA (GB)**

(74) Representative: **Wharton, Peter Robert**
**Urquhart-Dykes & Lord Alliance House 29-31**
**Kirkgate**
**Bradford West Yorkshire, BD1 1QB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method for the quantitative measurement of fat concentration in aqueous emulsions such as dairy products and in particular milk, by the use of infra-red techniques.

It is conventional in the field of infra-red analysis of milk samples to measure the concentration of fat in the triglyceride carbonyl band at a wavelength of about 5.73 µm at which energy is absorbed at the ester linkages in the fat molecules. See, for example the thesis by John Shields submitted for the degree of Bachelor of Philosophy at the University of York, November 1975. This technique yields satisfactory results so long as there is little variation in molecular weight among the various samples. However, genetic variations among cow breeds, and the practice of employing a variety of special feed stuffs, such as protected tallow, have caused sufficient variation in mean molecular weight of milk fat amongst differing herds that significant errors result when fat concentration is measured at the ester linkage absorption band. Another source of error is the substantial variation in mean molecular weight of dairy fat owing to seasonal changes in feed patterns.

It has also been proposed that the concentration of oily constituents in an aqueous sample, of which milk is an example, may be quantitatively measured employing infra-red absorption techniques at the absorption band associated with stretching of the saturated carbon hydrogen bond within the fat molecules (about 3.48 µm).

An instrument sold under the designation 'Oili' oil polution and monitoring system by Sahkoliikkeiden Oy of Finland since the middle 1970's employs this wavelength. However, measurement of infra-red absorption at the carbon hydrogen stretching band is subject to substantial variation due to variations in all natural fats, including milk or dairy fat, in molecular weight, degree of unsaturation and the nature of any side chains on the fatty acid, for example hydroxyl and methyl groups.

More recently, in European patent publication No. 0012492, it has been proposed to use infra-red analysis techniques in the 3.4 µm band for the analysis of milk samples. Both CH3 and CH2 groups absorb in this region of the spectrum. Most fatty acids have only one CH3 group per molecule so that the absorption by this is proportional to the number of triglycerides. Fatty acids with more than one methyl group will be over estimated. The absorption by CH2 groups is proportional to the number of CH2 groups and therefore is very nearly proportional to chain length. However, the presence of side chains, double bonds, or other substituents on the chain alters the characteristic absorption wavelength of adjacent CH2 groups so any of these factors will lead to under estimation of the amount of fat present. Consequently measurement at the CH stretching band has not in practice necessarily produced improved results because of variations in molecular weight, number of hydroxyl and methyl groups, and because the absorption into carbon hydrogen stretching region varies greatly with the protein and lactose concentrations present in milk samples. In particular, significant errors have been noted employing this technique with skimmed milk samples. Furthermore the use of the saturated carbon hydrogen stretching absorption band is of little merit when applied to synthetic dairy products prepared with polyunsaturated vegetable oils or to other types of natural oils which exhibit wide variations in both molecular weight and degree of unsaturation. Measurement at the 3.4 µm band is made difficult by the low energy available for detection owing to the strong water absorbence at this wavelength.

Greater accuracy of determination of fat in milk samples can be obtained by using measurements taken at both the 3.4 µm and the 5.73 µm band, as disclosed by Shields and Biggs. While this method has resulted in advantages when testing samples containing widely varying types of fat, for example in ice cream mixes, vegetable fat filled milks, and baby foods, it does take extra time in operation which may be disadvantageous to the user.

FR—A—2050525 discloses a method of determining the fat content of a milk sample using a reflectance method employing wide-band infra red radiation between 5 and 7 µm. Such a method does not have the accuracy of absorbence (transmission) measurements. US—A—3839633 discloses a double-beam absorption method of measuring, inter alia, fat in milk samples employing the previously favoured 5.7 µm band.

The present invention seeks to provide a method of quantitative measurement of fat concentration in aqueous emulsions in which the above disadvantages are eliminated or substantially reduced.

According to the present invention there is provided a method for quantitative determination of fat in an aqueous fat emulsion sample by an infra red transmission absorption technique characterised in that the infra red absorption of the sample is determined in a band having a wavelength of from 6.75 to 7.1 µm.

The waveband about 3.4 µm discussed hereinabove is characteristic of the stretching of carbon hydrogen bonds within a sample. We have unexpectedly found that by choosing a waveband which is characteristic of bending and scissoring instead, variations introduced by side chains or substituents on the fatty acid chains are substantially reduced. There are several wavebands associated with bending or scissoring of carbon hydrogen bonds but these are mainly at wavelengths below 3 µm. For reasons which will become more apparent hereinafter it is preferred to choose, if possible, a higher wavelength and the preferred wavelength to be used in the method of the present invention is the waveband about 6.84 µm.

Known apparatus for the infra red determination of fat in a sample, for example a milk sample, may use either a single wavelength dual cuvette or a double wavelength single cuvette system. The double wavelength system has several advantages, especially that it balances variations in water content of the

**0 122 749**

sample, compensates for scatter effects in the sample and compensates for accumulated dirt in the cuvette. It is therefore preferred to use such a double wavelength system, for example the system employed in the 'Multispec'® instrument available from Multispec Limited or Multispec Incorporated. When using such a double wavelength system, a measurement is taken at the 'sample' wavelength, for example in the band about 6.84 µm, and a measurement is taken at an adjacent wavelength at which the sample under test absorbs less strongly. The considerations involved in choosing reference wavelengths are discussed in the above mentioned thesis by John Shields, and the 'Multispec' instrument is discussed in US—A—4310763.

A further advantage of employing the wavelength about 6.84 µm is that the problems of light scatter from the fat globules in emulsions are considerably reduced in comparison with the 3.4 µm waveband. It is known that light scattering effects are related to the globule size in relation to the wavelength chosen; consequently at higher wavelengths higher globule sizes are tolerable. Accordingly, at the preferred wavelength of the invention, no special steps need be taken to reduce fat globule size below that employed in hitherto available apparatus and indeed homogenisation of fat containing samples need not be carried out to the same extent as hitherto.

Lipolysis of triglycerides, which increases with the age of a sample, causes a reduction in the number of carbonyl ester bonds in the sample. Since the carbonyl of the free fatty acids so produced has a different absorption wavelength from the carbonyl ester bond, measurements at 5.73 µm show an underestimation of fat as the degree of lipolysis increases. This is a particular problem when analysing composite samples, or poorly stored samples. Since the method of the invention does not involve measurement of carbonyl bonds it is not subject to these errors.

A further advantage of using the 6.84 wavelength band is that there is less absorbance due to water in the emulsion than is the case at the 3.4 µm band. Thus more energy passes through the sample and the sensitivity of measurement is increased when compared to the 3.4 µm band.

The invention will be illustrated further, by way of example only, with reference to the following Examples taken in conjunction with the accompanying drawings, in which:

Figure 1 is an infra-red absorption difference spectrum of milk against water;

Figure 2 shows spectra of lactose solution against water and protein solution/suspension against water;

Figure 3 is a similar spectrum to Figure 1 illustrating a different reference filter wavelength;

Figure 4 is a spectrum of atmospheric water vapour;

Figure 5 are spectra of homogenised and unhomogenised milk samples; and

Figure 6 shows three graphs illustrating the relative absorption of three wavelengths in comparison with fat chain length.

Example 1

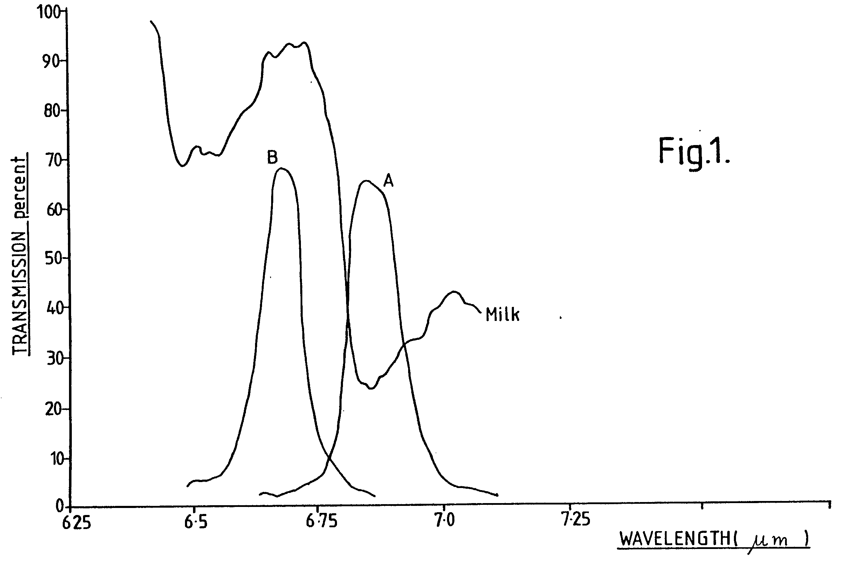

A standard Multispec milk analyser instrument was fitted with two filters A and B (see Figure 1), the sample filter having transmission characteristics in the waveband about 6.84 µm and the reference filter, filter B, having transmission in the band about 6.67 µm. Figure 1 also shows the absorption difference spectrum of a milk sample with respect to water in the range 6.25 to 7.25 µm. It can be seen that the absorption of the milk sample at the wavelength of filter A is very much greater than that of filter B enabling comparative measurements to be obtained.

The instrument was first linearised in the normal manner. A correlation coefficient of .99997 over the range 0% to 10% milk fat was achieved when comparing uncorrected instrument results against a linear dilution series of samples. The instrument was then calibrated in the normal way using the multiple regression programmes built into existing software.

The regression programme determines a slope value for the signal obtained from the filter pair and derives interference coefficients due to the other components in the mixture which, when introduced in the following algorithm (Equation 1) enable the instrument to quantify fat.

$$\text{Fat}_{est} = \text{Fat}_{inst} \times \text{slope} + (\text{protein}_{inst} \times \frac{p}{f}) + (\text{lactose}_{inst} \times \frac{l}{f}) \qquad \text{Equation 1}$$

$\frac{p}{f}$ = protein on fat correction coefficient

$\frac{l}{f}$ = lactose on fat correction coefficient

$\text{Fat}_{inst}$, $\text{Protein}_{inst}$, $\text{Lactose}_{inst}$ = uncorrected fat, protein, and lactose instrument results

$\text{Fat}_{est}$ = calculated fat concentration

The results of the above calibration are shown in Table 1.

3

**0 122 749**

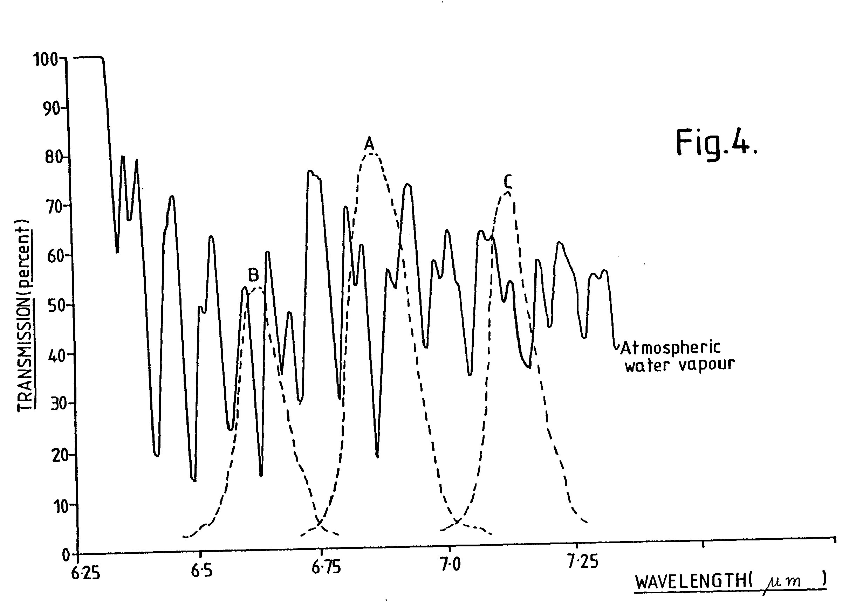

Referring to Figure 4, it will be observed that there is a good balance between water vapour absorbance at filter A and filter B transmission envelopes. This was verified in practice as drying and wetting of the instrument caused negligible change in response.

TABLE 1

1. Equation used

$$Fat_{est}=Fat_{inst}\times1.355+(protein_{inst}\times0.581)+(Lactose_{inst}\times-0.871)$$

2. Estimated values and difference

| Sample number | Sample type | Chemical value (C) | Estimated value (E) | Difference C−E |
|---|---|---|---|---|
| 1 | Milk | 3.94 | 3.94 | .0026 |
| 2 | Skimmed milk | 0.11 | 0.12 | −.0094 |
| 3 | Milk | 4.03 | 4.00 | .0332 |
| 4 | Milk | 4.61 | 4.62 | −.0062 |
| 5 | High fat milk | 5.50 | 5.52 | −.0205 |
| 6 | Lactose soln. | 0.00 | 0.00 | −.0002 |

3. Standard deviation of difference (S D Accuracy)=0.0182

4. Probable precision=0.0169

Example 2

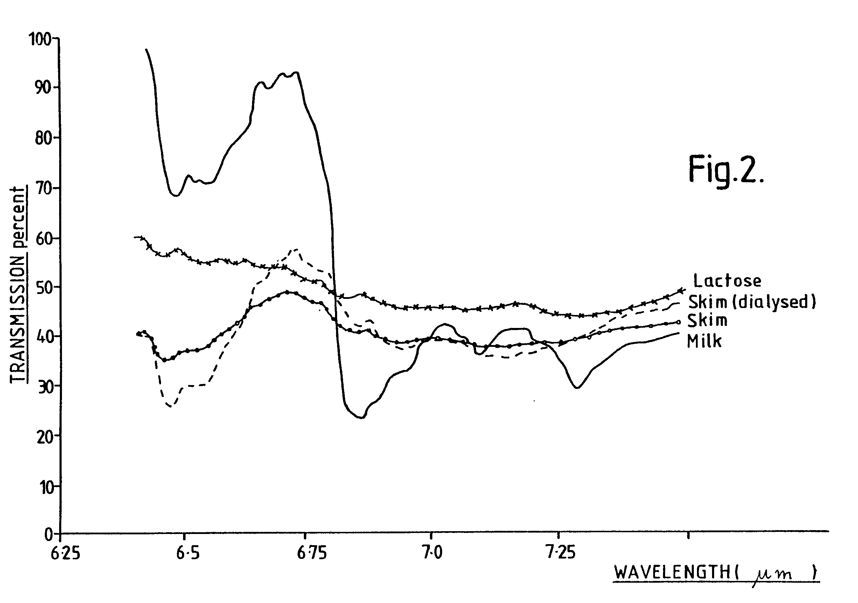

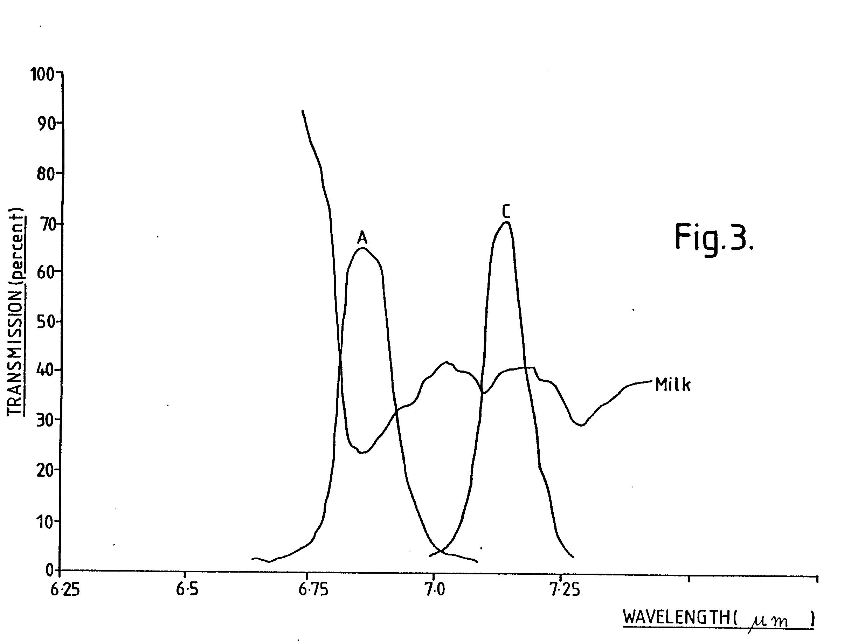

From the spectra illustrated in Figure 2 it is apparent that good results should be obtained by selecting a reference filter at about the 7.1 µm band. Accordingly, a 'Multispec'® instrument was provided with a sample filter, filter A, as described in Example 1 and a reference filter, filter C, having a transmission characteristic at about 7.12 µm. As before the instrument was linearised and calibrated in the normal way. Figure 3 illustrates the wavelengths of the sample and reference filters, and the good results were obtained with this reference wavelength also.

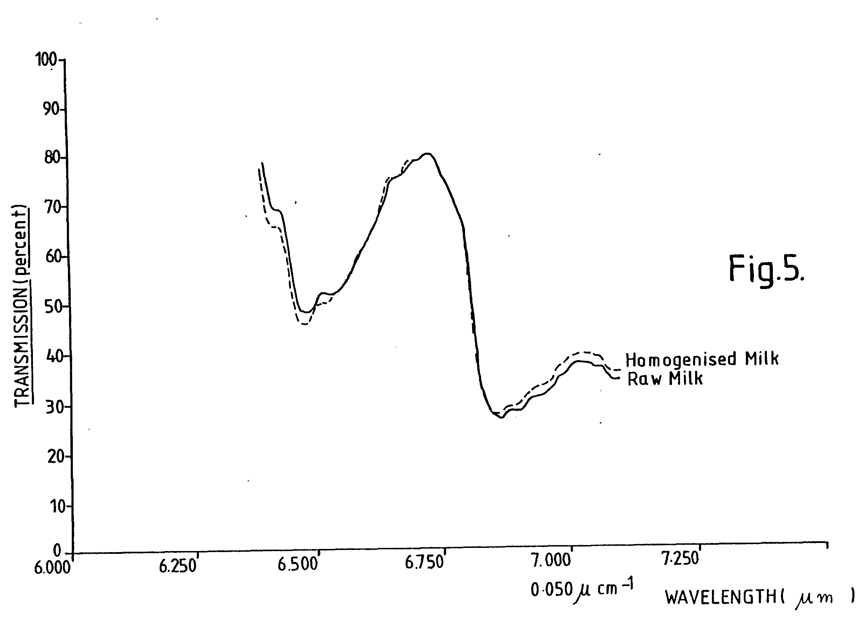

Figure 5 illustrates different spectra for raw milk and homogenised milk against water in the 6.25 to 7.25 µm wavelength range from which it can be seen that a correlation between the two is close. Table 3 below shows the effect of degree of homogenisation (as measured by homogenisation pressure in the Multispec instrument homogeniser) against the sample wavelength chosen. It can be seen from Table 3 that the accuracy of measurement at both 3.48 µm and 5.73 µm starts to fall off radically at the lowest homogenisation pressure whereas at the preferred wavelength of the present invention little error is introduced.

Table 2 below illustrates the effect of lipolysis as a function of sample wavelength chosen. The degree of lipolysis is proportional to the age of the sample and it can be seen that at both 5.73 µm and 3.48 µm that as the degree of lipolysis increases beyond a certain point errors are introduced whereas at the preferred 6.84 waveband such errors are minimised.

Reference has been made in the specification to the '6.84 µm waveband'. By this is meant generally the band from 6.75 to 7.10 µm, preferably with a centre about 6.84 µm. As is known in the infra red spectrometry field, for example as discussed in the Shields thesis referred to above, the band width of the filter chosen can be varied. Filters having band widths of 110 nm or less have been found to be suitable. As for the reference wavelengths, a reference wavelength in the range of 6.46 to 6.75 µm has been found suitable as has a reference wavelength in the range 7.0 to 7.20 µm, particularly 7.11 to 7.15 µm.

As discussed above homogenisation is less critical at the preferred wavelength of the present invention as the Christiansen effect, which is a change in apparent refractive index for a given wavelength, is reduced relative to measurement at the 3.4 µm and 5.73 µm waveband. However, it is preferred that the average fat globule diameter in the emulsion should be no greater than 3.5 µm.

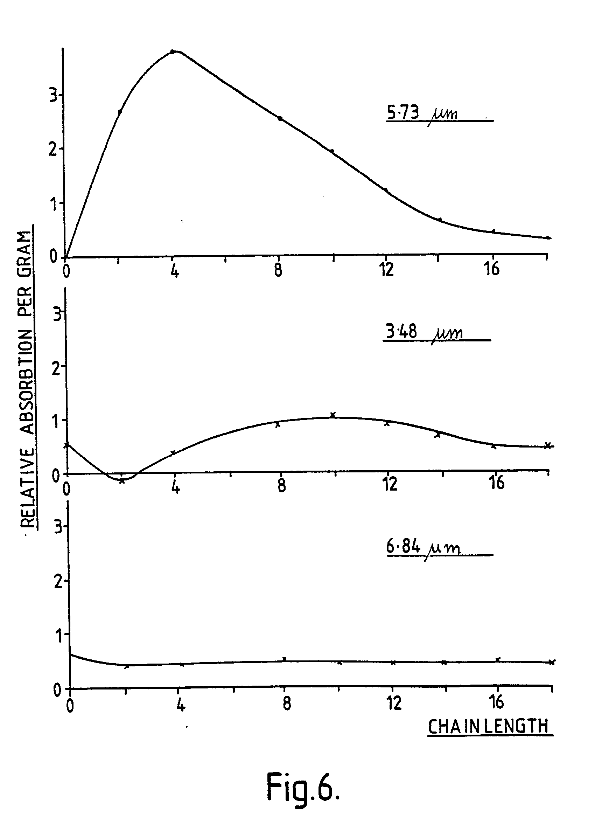

Referring to Figure 6 measurements were made of triglycerides having varying chain lengths at each of the two previously used wavelengths and the preferred wavelength of the present invention. The relative absorption per gramme at each wavelength was plotted against chain length of triglyceride sample. It can be seen that the variation at the 5.73 µm sample wavelength is considerable, as is the variation at the 3.4 µm waveband. However, the relative absorption of the 6.84 µm waveband is virtually constant irrespective of chain length. Thus it can be seen that errors arising through chain length variation in fat samples will be virtually eliminated using the preferred wavelength of the present invention.

4

**0 122 749**

A further advantage of the preferred waveband of the present invention is that an instrument measuring C-H can now employ a single blocking infra-red filter over the source of infra-red instead of each sample and/or reference filter having its own blocking element, as is present practice. This enables the cost of the instrument to be reduced.

TABLE 2

Showing the effect due to lipolysis (lipase on triolein) on Multispec
uncorrected readings at various wavelengths

| Wavelength Time (mins) | 5.73 | 6.84 | 3.48 |
|---|---|---|---|
| 0 | 5.16 | 2.25 | 3.97 |
| 10 | 5.21 | 2.30 | 4.13 |
| 20 | 5.18 | 2.28 | 4.12 |
| 30 | 4.99 | 2.29 | 3.75 |
| 40 | 4.97 | 2.30 | 3.79 |
| 50 | 4.66 | 2.14 | 3.72 |
| 60 | 4.49 | 2.22 | 3.39 |

TABLE 3

Showing the effect of homogenisation pressure Multispec uncorrected
readings of various wavelengths

| Wavelength µm Pressure lbs sq in | 5.73 | 6.84 | 3.48 |
|---|---|---|---|
| 2800 | 6.77 | 6.08 | 6.57 |
| 2700 | 6.78 | 6.06 | 6.59 |
| 2600 | 6.76 | 6.10 | 6.56 |
| 2400 | 6.71 | 6.06 | 6.53 |
| 2300 | 6.70 | 6.04 | 6.54 |
| 2100 | 6.31 | 6.04 | 5.88 |

**Claims**

1. A method for the quantitative determination of fat in an aqueous fat emulsion sample by an infra red transmission absorption technique characterised in that the infra red absorption of the sample is determined in a band having a wavelength of from 6.75 to 7.1 µm.

2. A method as claimed in claim 1 in which the wavelength is about 6.84 µm.

3. A method as claimed in either of claims 1 to 2 in which the infra red absorption technique is the double wavelength single cuvette system.

4. A method as claimed in claim 3 in which a reference wavelength is chosen in the range of 6.46 to 6.75 µm.

5. A method as claimed in claim 3 in which a reference wavelength is chosen in the range of 7 to 7.20 µm.

6. A method as claimed in claim 5 in which a reference wavelength is chosen in the range 7.11 to 7.15 µm.

7. A method as claimed in any one of claims 1 to 6 in which filters are employed to select the relevant wavelength and the filters have a band width of 110 nm or less.

8. A method as claimed in any one of claims 1 to 7 in which the fat globule diameter in the emulsion is no greater than 3.5 µm.

## Patentansprüche

1. Verfahren zur quantitativen Bestimmung von Fett in einer wässrigen Fettemulsionsprobe durch eine Infrarot-Transmissionsabsorptionstechnik, dadurch gekennzeichnet, daß die Infrarotabsorption der Probe in einem Band mit einer Wellenlänge von 6,75 bis 7,1 µm bestimmt wird.

2. Verfahren nach Anspruch 1, bei dem die Wellenlänge ungefähr 6,84 µm beträgt.

3. Verfahren nach Anspruch 1 oder 2, wobei die Infrarotabsorptionstechnik ein Doppelwellenlängen-Einfachküvettensystem ist.

4. Verfahren nach Anspruch 3, wobei eine Referenzwellenlänge im Bereich von 6,46 bis 6,75 µm gewählt wird.

5. Verfahren nach Anspruch 3, wobei eine Referenzwellenlänge im Bereich von 7 bis 7,20 µm gewählt wird.

6. Verfahren nach Anspruch 5, wobei eine Referenzwellenlänge im Bereich 7,11 bis 7,15 µm gewählt wird.

7. Verfahren nach einem der Ansprüche 1 bis 6, wobei Filter verwendet werden, um die relevante Wellenlänge zu wählen, und die Filter eine Bandbreite von 110 nm oder weniger haben.

8. Verfahren nach einem der Ansprüche 1 bis 7, wobei der Fettkügelchendurchmesser in der Emulsion nicht größer als 3,5 µm ist.

## Revendications

1. Méthode pour la détermination quantitative des matières grasses dans un échantillon d'émulsion aqueuse de graisse par une technique d'absorption de tranmission de rayonnement infrarouge, caractérisée en ce que l'absorption infrarouge de l'échantillon est déterminée dans une bande ayant une longueur d'onde de 6,75 à 7,1 µm.

2. Méthode suivant la revendication 1, dans laquelle la longueur d'onde est de 6,84 µm environ.

3. Méthode suivant la revendication 1 ou 2, dans laquelle la technique d'absorption de rayonnement infrarouge est le système à double longueur d'onde et cuvette unique.

4. Méthode suivant la revendication 3, dans laquelle une longueur d'onde de référence est choisie dans la plage de 6,46 à 6,75 µm.

5. Méthode suivant la revendication 3, dans laquelle une longueur d'onde de référence est choisie dans la plage de 7 à 7,20 µm.

6. Méthode suivant la revendication 5, dans laquelle une longueur d'onde de référence est choisie dans la plage de 7,11 à 7,15 µm.

7. Méthode suivant l'une quelconque des revendications 1 à 6, dans laquelle on emploie des filtres pour choisir la longueur d'onde correspondante et les filtres ont une largeur de bande de 110 nm ou moins.

8. Méthode suivant l'une quelconque des revendications 1 à 7, dans laquelle le diamètre des globules de graisse dans l'émulsion n'est pas supérieur à 3,5 µm.

Fig.1.

0 122 749

Fig.2.

Fig.3.

Fig.4.

TRANSMISSION (percent)

WAVELENGTH( μm )

Atmospheric water vapour

Fig.5.

Homogenised Milk
Raw Milk

TRANSMISSION (percent)

WAVELENGTH( $\mu m$ )

0·050 $\mu$ cm$^{-1}$

0 122 749

Fig.6.